# EUROPEAN PATENT APPLICATION

(11) **EP 1 798 802 A1**
(43) Date of publication of application: **20.06.2007**
(21) Application number: 05755722.5
(22) Date of filing: 29.06.2005
(51) Int. Cl.: H01M 10/40, C12P 19/04, H01B 1/06

(54) **LITHIUM ION CONDUCTIVE MATERIAL UTILIZING BACTERIAL CELLULOSE ORGANOGEL, LITHIUM ION BATTERY UTILIZING THE SAME AND BACTERIAL CELLULOSE AEROGEL**

(30) Priority: 30.08.2004 JP 2004250716
(71) Applicant: Nihon University, Tokyo 102-8275 (JP)
(72) Inventor: YANO, Shoichiro, Nihon University, Tokyo 1028275 (JP); SAWAGUCHI, Takashi, Nihon University, Tokyo 1028275 (JP); HAGIWARA, Toshiki, Nihon University, Tokyo 1028275 (JP); MAEDA, Hideaki, Kobe-shi, Hyogo 6550853 (JP); NAKAJIMA, Megumi, 3420033 (JP); SASAKI, Kazuhiro, 1200014 (JP)
(74) Representative: Epping, Wilhelm
(86) International application number: PCT/JP2005/011978
(87) International publication number: WO 2006/025148

(57) **Abstract**

A lithium ion conductive material that excels in mechanical strength, exhibiting high ion conductivity; a bacterial cellulose composite material having an inorganic material and/or organic material incorporated therein; and a bacterial cellulose aerogel. The water of bacterial cellulose hydrogel is replaced by a nonaqueous solvent containing a lithium compound. Bacterial cellulose producing bacteria are grown in a culture medium having an inorganic material and/or organic material added thereto. The bacterial cellulose hydrogel is dehydrated and dried.

## Description

### Technical Field

The present invention relates to an organic gel of bacterial cellulose (hereinafter also referred to as "bacterial cellulose organogel"), a lithium ion conductive material utilizing the same, a production method thereof, and a lithium ion battery using the same. The present invention also relates to a bacterial cellulose aerogel, a production method thereof, and a novel composite material using the same and a production method thereof.

### Background Art

Various kinds of batteries have been provided for practical uses to date, lithium ion batteries are drawing attention to deal with wireless of electronic devices because of light weight and capability of high electromotive force and high energy with less self-discharge as well. In particular, with increasing demands of further weight saving and less thickness in recent years, practical use of lithium ion battery using a polymer electrolyte instead of conventional electrolytes has been urged. Since such lithium ion battery has less leakage of electrolyte compared to batteries of conventional electrolytes, laminate resin films having aluminum thin membrane can be used as an exterior part in place of conventional metal cans, thereby producing a thin type battery with flexibility, thus there have been researched and developed lithium ion batteries using various polymer electrolytes (e.g., see Patent reference 1).

Polymer electrolytes are broadly classified into two types: a so called physical gel where linear polymer chains are entangled three dimensionally, namely, electrolyte is carried in a matrix composed of physically crosslinked polymers; and a so called chemical gel where electrolyte is carried in a matrix composed of chemically crosslinked polymers. In the case where a battery is produced with a chemical gel, for example, crosslinked polymers are formed in a battery container, i.e., by polymerization of monomer in situ to give a chemical gel simply and advantageously, however, unreacted monomer and polymerization initiator are left in electrodes and separators of battery to cause a drawback giving undesired influences to battery
characteristics. In the case where a battery is produced with a physical gel, polymer concentration in electrolyte must be increased to provide the physical gel with a suitable mechanical strength, also, if polymer concentration is not increased, a polymer with high molecular weight must be used, in this case, it becomes necessary to dissolve the polymer in an electrolyte under heating, which also requires a lot of time. Moreover, there arises a problem of deterioration of electrolyte salt due to heating.

Cellulose is a main component of plant cell wall, as a raw material of paper pulp, cellulose of wood being a higher plant is utilized through cooking and bleaching. Producing cellulose is not only a higher plant, but also bacteria, seaweed and ascidiacea are known as other cellulose source, since A.J. Brown reported that some kind of acetic acid bacteria formed cellulose membranes in a culture containing hydrocarbons, this system drew attentions as a biosynthesis model and has been researched. As a result, it was observed that bacterial cellulose produced by acetic acid bacteria was excreted outside of the bacteria as the pure cellulose, and a network structure of ribbon-like microstructures of several ten nm in width was formed as the shape, which was shown to be extremely fine in comparison with pulp fiber.
Also, as the features, there have been known fine structures, high cellulose content, high Young's modulus, and high biodegradability. The utilization is limited mainly to high value-added products. For example, specifically, Japanese Unexamined Patent Publication Shou 59-120159 discloses a medical pad, Japanese Unexamined Patent Publication Shou 61-281800 discloses an acoustic diaphragm, and Japanese Unexamined Patent Publication Shou 62-36467 discloses a molding material with high mechanical strength.
Patent reference 1: Japanese Unexamined Patent Publication 2003-317695
Patent reference 2; Japanese Unexamined Patent Publication Shou 59-120159 (1984)
Patent reference 3; Japanese Unexamined Patent Publication Shou 61-281800 (1986)
Patent reference 4: Japanese Unexamined Patent Publication Shou 62-36467 (1987)

### Disclosure of the Invention

### Problems to be solved by the Invention

The present invention provides a lithium ion conductive material as a novel material utilizing organic gel of bacterial cellulose. The present invention further provides a production method thereof and a lithium ion battery using the same. The present invention also provides a bacterial cellulose aerogel, a production method thereof and a novel composite material using the same. Further, the present invention provides a bacterial cellulose hydrogel, a composite material using a bacterial cellulose aerogel, and a production method thereof.

### Means to solve the Problems

The present inventors have keenly studied for finding excellent lithium ion conductive materials free from the above drawbacks of conventional lithium ion conductive materials on the basis of novel material, as a result, found the following and achieved the present invention: water in a bacterial cellulose hydrogel produced by acetic acid bacteria is completely replaced by a nonaqueous organic solvent containing a lithium ion to give an organic gel of bacterial cellulose containing a lithium ion, further, the resultant gel has excellent lithium ion conductivity.

Also, they have found that water in a bacterial cellulose hydrogel can be completely dried without deteriorating the shape using a solvent in a supercritical state and completed the present invention.

Namely, the present invention relates to a lithium ion conductive material wherein water in a bacterial cellulose hydrogel is replaced by a nonaqueous solvent containing a lithium compound.

Also, the present invention relates to a lithium ion conductive material wherein the nonaqueous solvent is selected from the group consisting of polyethylene glycol dimethyl ether, polyethylene glycol diethyl ether, polyethylene glycol dimethacrylate, polyethylene glycol diacrylate, polypropylene glycol dimethacrylate, and polypropylene glycol diacrylate.

Also, the present invention relates to a lithium ion conductive material wherein the nonaqueous solvent is particularly polyethylene glycol dimethyl ether.

Further, the present invention relates to a lithium ion conductive material wherein the lithium compound is selected from the group consisting of lithium perchlorate (LiClO₄), lithium borate tetrafluoride (LiBF₄), lithium phosphate hexafluoride (LiPF₆), lithium methanesulfonate trifluoride (LiCF₃SO₃), and lithium bistrifluoromethanesulfonylimide (LiN(CF₃SO₂)₂).

Also, the present invention relates to a lithium ion conductive material wherein the lithium compound is particularly lithium trifluoromethanesulfoneimide.

Also, the present invention relates to a production method of a lithium ion conductive material, comprising the steps of immersing a bacterial cellulose hydrogel in a nonaqueous solvent containing a lithium compound; being allowed to stand for a certain time under a reduced pressure and heating; subsequently raising temperature and further being allowed to stand for a certain time under a reduced pressure.

Further, the present invention relates to a production method of a lithium ion conductive material, wherein the nonaqueous solvent is selected from the group consisting of polyethylene glycol dimethyl ether, polyethylene glycol diethyl ether, polyethylene glycol dimethacrylate, polyethylene glycol diacrylate, polypropylene glycol dimethacrylate, and polypropylene glycol diacrylate.

Also, the present invention relates to a production method of a lithium ion conductive material, wherein the nonaqueous solvent is polyethylene glycol dimethyl ether.

Also, the present invention relates to a production method of a lithium ion conductive material, wherein the lithium compound is selected from the group consisting of lithium perchlorate (LiClO₄), lithium borate tetrafluoride (LiBF₄), lithium phosphate hexafluoride (LiPF₆), lithium methanesulfonate trifluoride (LiCF₃SO₃), and lithium bistrifluoromethanesulfonylimide (LiN(CF₃SO₂)₂).

Further, the present invention relates to a production method of a lithium ion conductive material, wherein the heating temperature and standing time at the first step are 30 to 90°C and 12 to 36 hours, respectively; the heating temperature and standing time at the second step are 100 to 160°C and 12 to 36 hours, respectively.

Also, the present invention relates to a production method of a lithium ion conductive material, wherein the heating temperature and standing time at the first step are 60°C and 24 hours, respectively; the heating temperature and standing time at the second step are 130°C and 24 hours, respectively.
Also, the present invention relates to a lithium ion battery including a cathode, an anode and the lithium ion conductive material of the present invention being disposed between the cathode and the anode.

Also, the present inventors have keenly studied for practical use of composite material of bacterial cellulose in the light of problems from viewpoints of mechanical, electrical characteristics and environmental protection even though various polymer composite materials in which inorganic substances are mixed and dispersed have been developed, as a result, they have found a production method of a composite material that various inorganic substances and organic polymer substances are dispersed in a bacterial cellulose hydrogel. Namely, the present inventors have found the following by culturing bacterial cellulose producing bacteria in a culture condition not known to date and have completed the present invention: various inorganic materials and/or organic materials can be incorporated in a bacterial cellulose hydrogel, further, the resultant bacterial cellulose hydrogel in which inorganic materials and/or organic materials are incorporated is subjected to treatments like dehydration to give a bacterial cellulose composite material in which inorganic materials and/or organic materials are incorporated.

Hereinafter, the bacterial cellulose composite material in which inorganic materials and/or organic materials are incorporated of the present invention includes a bacterial cellulose hydrogel in which inorganic materials and/or organic materials are incorporated, or one that a part of the water is eliminated, or one that almost all of the water is eliminated.

Namely, the present invention provides a composite material with totally new functions applicable to wide technical fields, and relates to a bacterial cellulose composite material in which inorganic materials and/or organic materials are incorporated.

Also, the present invention relates to a composite material wherein the inorganic material and/or the organic material are silica gel, silas balloon, carbon nanotube and/or polyvinyl alcohol, hydroxypropylcellulose.

Further, the present invention relates to a production method of a bacterial cellulose composite material wherein an inorganic material and/or an organic material are incorporated, wherein a bacterial cellulose producing bacterium is cultured in a culture medium added with an inorganic material and/or an organic material.

Also, the present invention relates to a production method of a bacterial cellulose composite material wherein an inorganic material and/or an organic material are incorporated, wherein in the culture medium, as a carbon source, glucose, mannitol, sucrose, maltose, hydrolyzed starch, molasses, ethanol, acetic acid, or citric acid is used; as a nitrogen source, ammonium salt such as ammonium sulfate, ammonium chloride, and ammonium phosphate, nitrate, urea, or polypeptone is used; as inorganic salts, phosphate, calcium salt, iron salt or manganese salt is used; and as an organic trace nutrient, amino acid, vitamin, fatty acid, nucleic acid, casamino acid, yeast extract, or hydrolyzed soy protein is used.

Also, the present invention relates to a production method of a bacterial cellulose composite material wherein an inorganic material and/or an organic material are incorporated, wherein the culture medium includes glucose, polypeptone, yeast extract, and mannitol.

Also, the present invention relates to a production method of a bacterial cellulose composite material wherein an inorganic material and/or an organic material are incorporated, wherein the bacterial cellulose producing bacterium is a microbe belonging to Acetobacter, Gluconobacter, Agrobacterium or Pseudomonas.

Further, the present invention relates to a production method of a bacterial cellulose composite material wherein an inorganic material and/or an organic material are incorporated, wherein the bacterial cellulose producing bacterium is Acetobacter xylinum (IFO NO 13772).

Further, the present invention relates to a production method of a bacterial cellulose composite material wherein an inorganic material and/or an organic material are incorporated, wherein the bacterial cellulose producing bacterium is a new strain obtained from Acetobacter xylinum (IFO NO 13772), (National Institute of Advanced Industrial Science and Technology. International Patent organism Depositary, Depositary Number FERM P-20332, International Depositary Number FERM BP-10357).

Also, the present invention relates to a production method of a bacterial cellulose composite material wherein an inorganic material and/or an organic material are incorporated, wherein the inorganic material and/or the organic material are silica gel, silas balloon, carbon nanotube and/or polyvinyl alcohol, hydroxypropylcellulose.

The present inventors have keenly studied on a complete drying method with hardly deteriorating the shape of bacterial cellulose hydrogel, as a result, they have found that a certain solvent can be dried by adopting a supercritical condition.

Therefore, the present invention relates to a bacterial cellulose aerogel as a novel aerogel.

Also, the present invention relates to a production method of bacterial aerogel, wherein a bacterial cellulose hydrogel is dehydrated and dried with a supercritical ethanol.

Also, the present invention relates to a production method of bacterial hydrogel, wherein water or water containing a salt is absorbed in a bacterial cellulose aerogel.

Further, the present invention relates to a production method of bacterial cellulose organogel, wherein an organic solvent or a solvent containing a salt is absorbed in a bacterial cellulose aerogel.

Also, the present invention includes hydrogel and organogel obtained from a bacterial cellulose aerogel, and hydrogel and organogel containing various salts.

### Effect of the Invention

The lithium ion conductive material of the present invention, since water in a bacterial cellulose hydrogel is completely replaced by a nonaqueous solvent containing a lithium compound, has excellent lithium ion conductivity and exhibits excellent characteristic of mechanical strength. Lithium ion battery with excellent performance can be obtained by using the lithium ion conductive material having such characteristics as a separator.

According to the production method of the present invention, various inorganic materials and/or organic materials can be incorporated in bacterial cellulose fibers. Therefore, a composite material obtained by the production method of the present invention exhibits excellent moldability, mechanical and electrical characteristics, and biodegradability.

The bacterial cellulose aerogel of the present invention is a dried one with almost no change of the shape of bacterial cellulose hydrogel. Thus, various organic solvent as well as water can be contained therein with almost no limitation, which can prepare a hydrogel or an organogel. These become base materials for novel composite materials.

### Brief description of the Drawings

FIG. 1 shows comparative results on thickness of bacterial cellulose gels formed by mutants of Acetobacter xylinum.
FIG. 2 shows a Cole-Cole plot of PEO-BC gel electrolyte (measuring temperature of 55°C, thickness of 0.1076 cm, 9.78×10⁻³S/cm).
FIG. 3 shows the content of silica in the sample obtained in Example 7.
FIG. 4 shows the tensile test results of the sample obtained in Example 7.
FIG. 5 shows the DMA test results of the sample obtained in Example 7.
FIG. 6 shows an electron micrograph of silas balloon bacterial cellulose obtained in Example 8.
FIG. 7 shows the content of silas balloon in the sample obtained Example 8.
FIG. 8 shows the tensile test results of the sample obtained in Example 8.
FIG. 9 shows the DMA test results of the sample obtained in Example 8.
FIG. 10 shows an electron micrograph of carbon nanotube bacterial cellulose obtained in Example 9.
FIG. 11 shows the content of carbon nanotube in the sample obtained Example 9.
FIG. 12 shows the tensile test results of the sample obtained in Example 9.
FIG. 13 shows the DMA test results of the sample obtained in Example 9.
FIG. 14 shows the tensile test results of the sample obtained in Example 10.
FIG. 15 shows the DMA test results of the sample obtained in Example 10.
FIG. 16 shows the tensile test results of the sample obtained in Example 11.
FIG. 17 shows the DMA test results of the sample obtained in Example 11.
FIG. 18 shows an electron micrograph (10000 times) of bacterial cellulose aerogel obtained in Example 13.
FIG. 19 shows the compression test results of bacterial cellulose hydrogel, bacterial cellulose aerogel, bacterial cellulose polyethylene oxide gel, and bacterial cellulose xylene gel.
FIG. 20 shows infrared absorption spectra of bacterial cellulose PEO ether obtained in Example 18.
FIG. 21 shows the temperature dependence of lithium ion conductivity for bacterial cellulose PEO gel electrolyte (Mw 250 and Mw 550) and PEO-grafted bacterial cellulose solid electrolyte.
FIG. 22 shows infrared absorption spectra of bacterial cellulose PEO ester obtained in Example 20.

### Best mode carrying out the Invention

### (Lithium ion conductive material)

The lithium ion conductive material of the present invention is a bacterial cellulose organic gel, wherein water in a bacterial cellulose hydrogel is replaced by a nonaqueous solvent containing a lithium compound.

Components of bacterial cellulose hydrogel here used in the present invention are those produced by microbes, any one of cellulose, heteropolysaccharide with cellulose as a main chain, glucan such as β-1, 3 and β-1, 2, or mixtures thereof. Additionally, constitutional components other than cellulose in the case of heteropolysaccharide are 6C-sugars, 5C-sugars and organic acids such as mannose, fructose, galactose, xylose, arabinose, rhamnose, and glucuronic acid.

Also, bacterial cellulose hydrogel usable in the present invention has a very high mechanical strength in spite of gel substance (platy, lamellar) consisting of cellulose fiber and water. Such high mechanical strength of bacterial cellulose can be achieved by suitably adjusting culture conditions of acetic acid bacteria of producing bacteria and randomly entwining fibrous cellulose fibrils excreted from bacteria cell randomly moving around in culturing. Also, bacterial cellulose hydrogel usable in the present invention features a solid content contained in bacterial cellulose as low as 0.5 to 1.0% by weight in spite of the high mechanical strength.

Bacterial cellulose hydrogel usable in the present invention is not particularly limited as long as it is so called cellulose producing bacteria. Specifically, it includes acetic acid bacteria (Acetobacter) such as Acetobacter xylinum subsp. sucrofermentans typified by BPR2001 strain, Acetobacter xylinum ATCC23768, Acetobacter xylinum ATCC 23769, Acetobacter pasteurianus ATCC10245, Acetobacter xylinum (IFO NO 13772), Acetobacter xylinum ATCC14851, Acetobacter xylinum ATCC11142 and Acetobacter xylinum ATCC10821; in addition thereto, Agrobacterium, Rhizobium, Sarcina, Pseudomonas, Achromobacter, Alcaligenes, Aerobacter, Azotobacter and Zoogloea, and various mutant created by mutant treatment with known methods using NTG (nitroguanidine). Acetobacter xylinum (IFO NO 13772) is advantageous. Mutant of Acetobacter xylinum (IFO NO 13772) is more preferable.

Also, the shape of bacterial cellulose hydrogel usable in the present invention is not particularly limited. Preferable shapes (in cylindrical, platy and membranous cases, longitudinal, width, height and thickness; in discoid case, radius and thickness) can be freely produced by suitably choosing culture conditions and culture devices. Also, the resultant bacterial cellulose hydrogel can be cut as it is to a preferable shape. Specifically, there are listed platy, cylindrical, membranous, discoid, ribbon, cylindrical, and linear shapes.

Also, the bacterial cellulose hydrogel usable in the present invention can be generally stored for a long period of time by known storage methods. A storage stabilizer may be added if necessary.

A nonaqueous solvent by which water of the bacterial cellulose hydrogel usable in the present invention is replaced dissolves a lithium salt described below, is not particularly limited as long as it replaces water of the bacterial cellulose hydrogel completely without destroying its shape. Specifically, there are listed at least one selected from the group consisting of polyethylene glycol dimethyl ether, polyethylene glycol diethyl ether, polyethylene glycol dimethacrylate, polyethylene glycol diacrylate, polypropylene glycol dimethacrylate, and polypropylene glycol diacrylate, or mixtures thereof. Also, in the case of use for a lithium battery, a solvent that can stably stand up against electrochemical changes of lithium/lithium ion is preferred, for this purpose, polyethylene glycol dimethyl ether is particularly preferable.

Further, the lithium compound that is used together with a nonaqueous solvent by the present invention is not particularly limited as long as it dissolves sufficiently in the nonaqueous solvent and is present stably. Specifically, it is preferable to use at least one selected from the group consisting of lithium perchlorate (LiClO₄), lithium borate tetrafluoride (LiBF₄), lithium phosphate hexafluoride (LiPF₆), lithium methanesulfonate trifluoride (LiCF₃SO₃), and lithium bistrifluoromethanesulfonylimide (LiN(CF₃SO₂)₂). In the case of use for a lithium battery, a lithium compound that can stably stand up against electrochemical changes of lithium/lithium ion is preferred, for this purpose, lithium bistrifluoromethanesulfonylimide is particularly preferable.

Further, the content of lithium ion is also not particularly limited, suitable concentrations for utilizing organic gel of bacterial cellulose of the present invention can be prepared. Specifically, a range of 0 to 20 mol% based on EO unit is possible; in the case of use as a lithium ion conductor of lithium ion battery, a range of 5 to 6 mol% is possible.

The organic gel of bacterial cellulose of the present invention features very high mechanical strength in spite of very low solid content. Various measuring methods can be used for evaluating the mechanical strength of organic gel. Also, the organic gel of bacterial cellulose can be cut as it is to a preferable shape. Specifically, there are listed platy, cylindrical, membranous, discoid, ribbon, cylindrical, and linear shapes.

The organic gel of bacterial cellulose of the present invention contains a lithium ion, and the ion conductance can be evaluated in various measuring methods.

The lithium ion conductance of the organic gel of bacterial cellulose of the present invention containing a lithium ion depends on the kind of nonaqueous organic solvent, the kind and concentration of lithium ion contained, temperature, shape or the like.

The production method of the present invention is characterized in that water in a bacterial cellulose hydrogel is completely replaced by a nonaqueous solvent containing a lithium compound. Thus, it is not particularly limited as long as the method can substitute nonaqueous solvent molecule for water molecule in a bacterial cellulose hydrogel without largely deteriorating the shape and property of gel. Specifically, substitution can be done by immersing bacterial cellulose hydrogel in a nonaqueous solvent. Further, immersion can be conducted under a reduced pressure so that the substitution is performed rapidly and completely. Further, it can be conducted under a suitable heating condition. More specifically, it is preferable that a bacterial cellulose hydrogel is immersed in a nonaqueios solvent, and allowed to stand for a certain time under reduced pressure and heating conditions followed by raising temperature, further allowed to stand for a certain time under a reduced pressure. Here, the heating temperature and standing time at the first step are 30 to 90°C and 12 to 36 hours, preferably 50 to 70°C and 20 to 30 hours, particularly preferably 60°C and 24 hours, respectively. Also the heating temperature and standing time at the second step are 100 to 160°C and 12 to 36 hours, preferably 120 to 140°C and 20 to 30 hours, particularly preferably 130°C and 24 hours, respectively.

The lithium ion battery of the present invention is characterized by including a cathode, an anode and the lithium ion conductive material of the present invention being disposed between the cathode and the anode. Herein, the cathode and anode materials used in the present invention are not particularly limited, may be those used in generally known lithium ion batteries. In particular, a cathode material is LiMnO₂, LiCoO₂ or LiNiO₂. Also, as an anode material used in the present invention, it is a carbon material capable of storing/discharging lithium ions. The shape of lithium ion conductor of lithium ion battery of the present invention is also not particularly limited, various shapes such as platy, membranous and ribbon can be suitably chosen.

The bacterial cellulose composite material of the present invention is characterized by a structure that various inorganic materials and/or organic materials are incorporated in bacterial cellulose fibers. Herein, bacterial cellulose includes a water-containing hydrogel, one partly containing water, and one dehydrated and dried.

The kind, shape and content of inorganic material and/or organic material incorporated are not particularly limited. A preferable kind of inorganic material and/or organic material can be chosen for providing a composite material with desired characteristics. Specifically, there are listed an inorganic material such as silica gel, silas balloon, carbon nanotube, and an organic material such as polyvinyl alcohol and hydroxypropylcellulose. Also, as the shape (or size), materials with various shapes such as spherical, needle, rod, platy and irregular are incorporated. In particular, a material of nanometer size is incorporated in the composite of the present invention. The content is also not particularly limited, it can be suitably chosen to meet an intended use of composite material. The content of an inorganic material and/or an organic material is generally in a range of 1 to 25% by weight.

Further, the structure of composite of the present invention is not a conventionally known one that a cellulose material is merely mixed with an inorganic material and/or an organic material, but has a characteristic structure that an inorganic material and/or an organic material are almost uniformly dispersed in cellulose fibers. Such structure can be easily observed using an electron microscope for example.

Also, the composite of the present invention includes materials that various treatments are conducted to a bacterial cellulose hydrogel obtained by a culture method described below. Such treatments specifically include dehydrating/drying, compressing deformation, dehydrating compression drying, molding treatments; and dehydrating, drying, compressing deformation, dehydrating compression drying treatments after molding treatment. By performing such treatments, for example, a bacterial cellulose hydrogel is dried into flake so that the shape can be formed to be paper-like, platy and ribbon-like. Also, it can be formed into a preferable three-dimensional shape by compression dehydrating formation using a suitable mold. The composite thus formed can be preferably used for acoustic materials like speaker cone, dishware such as plate and cup, medical device materials, toy and stationary materials, building materials, clothing materials, interior materials in vehicle and house. Also, it is very excellent in biodegradability, and a material with good environmental suitability.

Physical properties of the composite of the present invention can be evaluated by using conventionally known various measuring methods for physical properties and the apparatuses. Also, for either a hydrogel state or a dried state, the evaluations can be done by using conventionally known various measuring methods for physical properties and the apparatuses. Moreover, for the treated and molded composites described above, the evaluations can be done by using various measuring methods for physical properties and apparatuses.
Specifically, mechanical strength characteristics can be evaluated by dynamic viscoelastic measurement and tensile test, further thermodynamic characteristics can be evaluated by thermal weight measurement.

The production method of the present invention is a method that can obtain bacterial cellulose hydrogel in which an inorganic material and/or an organic material are incorporated by culturing bacterial cellulose producing bacteria in a culture medium added with an inorganic material and/or an organic material under a specific culture condition.

The bacterial cellulose producing bacteria usable in the present invention is not particularly limited as long as it can produce cellulose in a culture, for example, there are listed microbes belonging to Acetobacter, Gluconobacter, Agrobacterium and Pseudomonas. Among them, microbe of Acetobacter is preferable, Acetobacter xylinum in particular, Acetobacter xylinum (IFO NO 13772) is advantageously preferable. Further a mutant of Acetobacter xylinum (IFO NO 13772) is preferable. Specifically, the mutant with the depositary number deposited in National Institute of Advanced Industrial Science and Technology is preferably used.

Culture components usable in the present invention may use a culture containing a carbon source, a nitrogen source, inorganic salts, organic trace nutrients such as amino acid and vitamin as well according to demand, as a carbon source, glucose, mannitol, sucrose, maltose, hydrolyzed starch, molasses, ethanol, acetic acid, or citric acid is used; as a nitrogen source, ammonium salt such as ammonium sulfate, ammonium chloride, and ammonium phosphate, nitrate, urea, or polypeptone is used; as inorganic salts, phosphate, calcium salt, iron salt or manganese salt is used; and as an organic trace nutrient, amino acid, vitamin, fatty acid, nucleic acid, casamino acid, yeast extract, or hydrolyzed soy protein is used. Glucose, polypeptone, yeast extract and mannitol are preferable.

An inorganic material and/or an organic material may be added in an arbitral point of time in culturing, it is preferable to be added before start of culturing. Culture conditions of the present invention are pH of 5 to 9, temperature of 10 to 40°C, particularly preferably under control of 25 to 30°C for 1 to 15 days, preferably about 1 to 3 days. As the culture states usable in the present invention, there are a still standing culture, through-flow stirring culture, shaking culture, vibrating culture and air-lift type culture, it is not particularly limited in the present invention, a still standing culture is preferable. The shape of container for culturing is also not particularly limited, it can be chosen for bacterial cellulose hydrogel to form in a desired shape.

### (Bacterial cellulose aerogel)

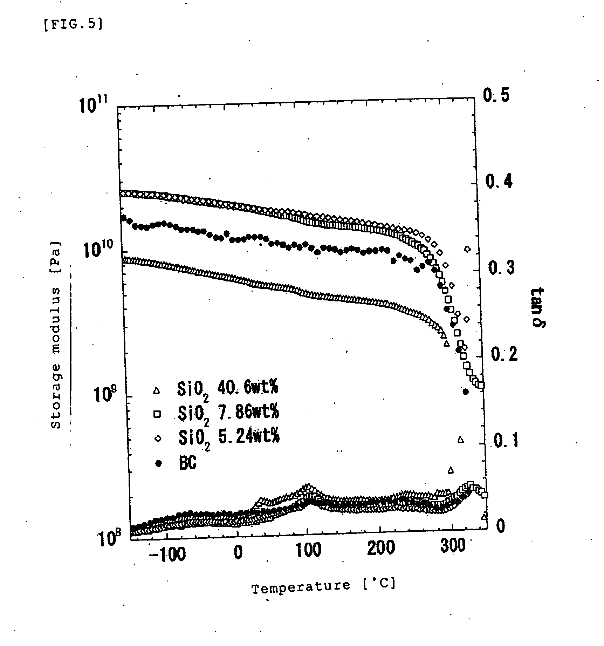

Also, the bacterial cellulose aerogel of the present invention is a novel aerogel obtained by drying bacterial cellulose hydrogel. The structure can be easily observed with an electron microscope. FIG. 5 shows an example. From the photograph, it is known that the inside of aerogel has a structure in which fine cellulose fibrils of several ten nm are highly branched in three dimensions. The drying method is not particularly limited, may be a means capable of dehydrating and drying without deteriorating the shape largely. For example, preferable is a method using supercritical methanol, ethanol, isopropanol, or isobutanol. Specifically, supercritical drying using ethanol is listed. In this case, pressure is preferably in a range of 6.38 to 11 MPa, and temperature is preferably in a range of 243 to 300°C.

The resultant aerogel hardly change in its shape. Thus the density is very low. It is about 6 mg/l. The bacterial cellulose aerogel of the present invention is white and somewhat transparent. It is apt to adhere to surfaces of various materials. It adheres easily on glass, metal, plastic, skin etc. The adhesion is not due to electrostatic. This fact is neither due to the residual surface water. It can be easily cut with a sharp cutter such as a knife.

When the bacterial cellulose aerogel of the present invention is immersed in water or other solvent, it can easily absorb the solvent. The solvent includes a polar organic solvent and nonpolar organic solvent in addition to water. Specifically, there are listed water, toluene, benzene, xylene, diethyl ether, ethyl acetate, acetone, methyl ethyl ketone, methanol, ethanol, isopropanol, isobutanol, polyethylene glycol dimethyl ether (Mw 250), polyethylene glycol (Mw 600), dimethyl sulfoxide, dimethylacetoamide, dimethylformamide, n-hexane, tetrahydrofuran, and silicone oil. The organogel containing the solvent maintains its shape. Further, when the organogel is lifted from a solvent, the solvent tends to be mostly held inside the gel while withstanding gravity. When the bacterial cellulose aerogel of the present invention is immersed in water or other solvent, it can simultaneously suck various salts present in the solvent. For example, for obtaining a lithium ion conductive material, as the solvent there are listed at least one selected from the group consisting of polyethylene glycol dimethyl ether, polyethylene glycol diethyl ether, polyethylene glycol dimethacrylate, polyethylene glycol diacrylate, polypropylene glycol dimethacrylate, and polypropylene glycol diacrylate, or mixtures thereof. Also, in the case of use for a lithium battery, a solvent that can stably stand up against electrochemical changes of lithium/lithium ion is preferable, for this purpose, polyethylene glycol dimethyl ether is particularly preferable. Also, as the lithium salt, specifically it is preferable to use at least one selected from the group consisting of lithium perchlorate (LiClO₄), lithium borate tetrafluoride (LiBF₄), lithium phosphate hexafluoride (LiPF₆), lithium methanesulfonate trifluoride (LiCF₃SO₃), and lithium bistrifluoromethanesulfonylimide (LiN(CF₃SO₂)₂). In the case of use for a lithium battery, a lithium compound that can stably stand up against electrochemical changes of lithium/lithium ion is preferable, for this purpose, lithium bistrifluoromethanesulfonylimide is particularly preferable.

### Examples

The present invention will be described in detail with Examples below. Additionally, the present invention is not to be limited to the examples.

### (Example 1) Preparation of bacterial cellulose:

### 1. Production of agar medium

In 100 ml of pure water were dissolved 0.5 g of glucose, 0.5 g of polypeptone, 0.1 g of magnesium sulfate, 0.5 g of yeast extract and 0.5 g of mannitol, to the solution, 2 g of agar was added and heated to dissolve. The resultant solution was divided into test tubes by 8 ml, sealed with an urethane culture-plug. The plug was further covered tightly with an aluminum foil. Heat sterilization was conducted in an autoclave at 120°C for 9 minutes. The sterilized solution was allowed to stand at a slant overnight, the generated gel was used as a slant culture.

### 2. Bacteria inoculation into culture

Acetobacter xylinum (FERM P-20332) was inoculated into the foregoing slant culture and cultured at 30°C.

### 3. Preparation of culture liquid

In 500 ml of pure water were dissolved 15 g of glucose, 2.5 g of polypeptone, 0.5 g of magnesium sulfate, 2.5 g of yeast extract and 2.5 g of mannitol, heat sterilization was conducted in an autoclave at 120°C for 9 minutes.

### 4. Preparation of mother liquid

The same solution as the culture liquid was prepared, of which about 5 ml was added to a test tube, bacteria were washed out from the slant culture. The liquid was brought back again in the culture liquid, and was allowed to stand at 30°C for 3 days to activate the bacteria to yield a mother liquid.

### 5. Culturing

The mother liquid and culture liquid were mixed in a ratio of 1:1, ethanol was added thereto to be 0.4% by weight, developed in a petri dish. This was still-cultured at 30°C for 25 days to give a bacterial cellulose gel.

### 6. Bleaching of bacterial cellulose gel

The generated gel was sufficiently washed with running water, immersed in 1 wt% aqueous sodium hydroxide for 24 hours, impurities such as microbes were dissolved to eliminate. Next, it was immersed in 0.5 wt% aqueous sodium hypochlorite for 12 hours to bleach, then washed sufficiently with running water to yield a bacterial cellulose sample.

### (Example 2) Mutant of Acetobacter xylinum

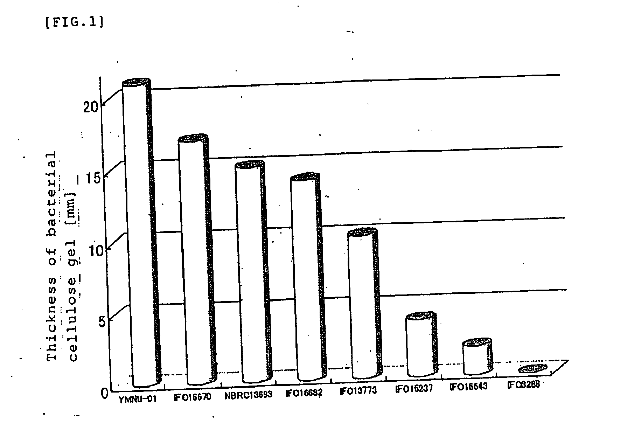

Acetobacter xylinum (IFO13772) was cultured in the same manner as in Example 1. The resultant Acetobacter xylinum was named YMNU-01 and deposited in the National Institute of Advanced Industrial Science and Technology (National Institute of Advanced Industrial Science and Technology, International Patent Organism Depositary, Depositary Number FERM P-20332, International Depositary Number FERM BP-10357). As shown in FIG. 1, it was known that the resultant Acetobacter xylinum generated a very thick gel.

### (Example 3) Production of bacterial cellulose gel electrolyte

### 1. Preparation of lithium ion electrolytic solution Lithium bistrifluoromethanesulfonylimide of 78.50 g was dissolved in polyethylene glycol dimethyl ether of 200 g to yield an electrolytic solution.

### 2. Preparation of gel electrolyte

In the above electrolytic solution prepared in a separable flask, the bacterial cellulose sample of 118 g was immersed, and was allowed to stand at 60°C under a reduced pressure for 24 hours to mix dispersion media. Next, temperature was raised stepwise, allowed to stand finally at 130°C under a reduced pressure for 24 hours to exchange dispersion media to yield a gel electrolyte.

### (Example 4) Measurement of lithium ion conductance of gel electrolyte

### 1. Impedance measurement

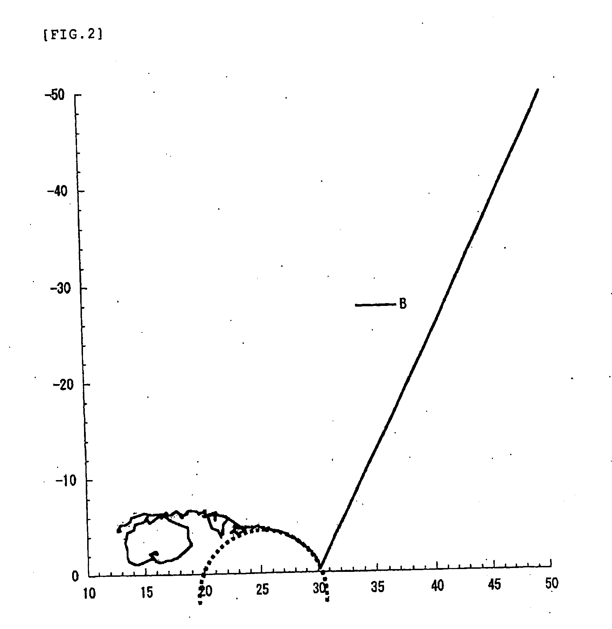

Impedance of a sample which was sandwiched with cupper plates of 23.5 mm in diameter was measured using an impedance measuring apparatus (PRECISION LCR METER 4284A model manufactured by HP Corporation), with an applied voltage of 10 mV, measuring frequency of 20 Hz to 1 MHz, at 30°C under a helium atmosphere. The sample measured was cylindrical having a diameter of 23.5 mm and a thickness of 3.29 mm. The resulting Nyquist plots are shown in FIG. 2. The ion conductivity of the gel electrolyte was 9.78×10⁻³ [S/cm] from them.

### (Example 5) Production of lithium ion battery

### 1. Preparation of cathode

Aqueous 5 wt% manganese (II) sulfate was prepared. A manganese oxide electrode was prepared by electrolyzing at a direct current voltage of 3V with a carbon rod as a positive electrode and a cupper plate as a negative electrode.

### 2. Preparation of anode

A lithium ribbon of 0.75 mm in thickness was cut to 20 mm×10 mm under a nitrogen atmosphere in a glove box to prepare an anode.

### 3. Production of battery and measurement of voltage

A bacterial cellulose sample of 5 mm in thickness was cut to 20 mm×20 mm, sandwiched between cathode and anode to prepare a lithium battery. The voltage of the battery was measured several times using a tester (DIGITAL MLTMETER CD 721 model), the voltage was determined to be 3.4 V in average.

### (Example 6) Production of composite material utilizing bacterial cellulose hydrogel

Herein an agar culture was produced as follows. In 100 ml of pure water were dissolved 0.5 g of glucose, 0.5 g of polypeptone, 0.1 g of magnesium sulfate, 0.5 g of yeast extract and 0.5 g of mannitol, to the solution, 2 g of agar was added and heated to dissolve. The resultant solution was divided into test tubes by 8 ml, sealed with an urethane culture-plug. The plug was further covered tightly with an aluminum foil. Heat sterilization was conducted in an autoclave at 120°C for 9 minutes. The sterilized solution was allowed to stand at a slant overnight, the generated gel was used as a slant culture.

Also, Acetobacter xylinum was inoculated into the slant culture and cultured at 30°C.

Also, a culture liquid was prepared as follows. In 500 ml of pure water were dissolved 15 g of glucose, 2.5 g of polypeptone, 2.5 g of yeast extract and 2.5 g of mannitol, heat sterilization was conducted in an autoclave at 120°C for 9 minutes.

Further, a mother liquid was prepared as follows. The same solution as the culture liquid was prepared, of which about 5 ml was added to a test tube, bacteria were washed out from the slant culture. The liquid was brought back again in the culture liquid, and was allowed to stand at 30°C for 3 days to activate the bacteria to yield a mother liquid.

### (Example 7) Production method of bacterial cellulose in which colloidal silica is incorporated

1. Culturing bacterial cellulose producing bacteria in the presence of colloidal silica (Snowtex O, Snowtex S, Snowtex 20 manufactured by Nissan Chemical Industries, Ltd.) Culture liquid of 80 ml, mother liquid of 100 ml, colloidal silica of 20 ml, mother liquid was 100 ml, culture liquid of 90 ml and colloidal silica of 10 ml; culture liquid of 95 ml and colloidal silica of 5 ml were mixed, and developed in petri dishes, then cultured for 25 days.

2. Bleaching of colloidal silica bacterial cellulose gel After 25 days, the generated gel was sufficiently washed with running water, next immersed in 0.5 wt% aqueous sodium hypochlorite for 12 hours to bleach, then washed sufficiently with running water to yield a colloidal silica bacterial cellulose sample.

3 Production of compressed film of colloidal silica bacterial cellulose sample
   The colloidal silica bacterial cellulose sample was pressed by a heat press at 120°C, at 1 to 2 MPa to yield a film.

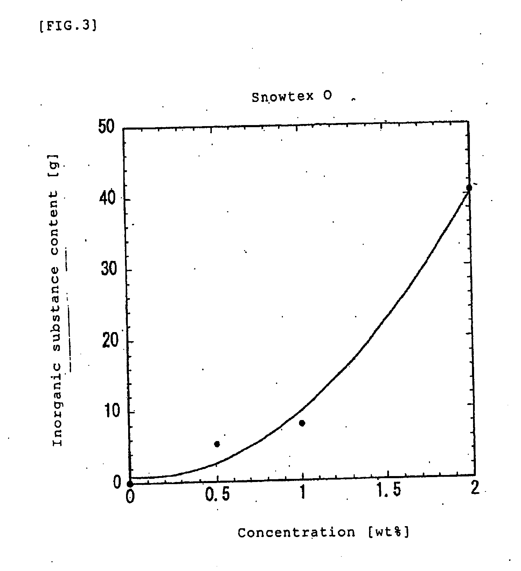

4. Measurement for presence of silica in colloidal silica bacterial cellulose sample
   About 100 mg of the dried film of colloidal silica bacterial cellulose sample was weighed out, heated at 900°C in an electric furnace for 3 hours, the content of inorganic component was estimated from the weight of ash. The results are shown in FIG. 3. This indicates the presence of silica.

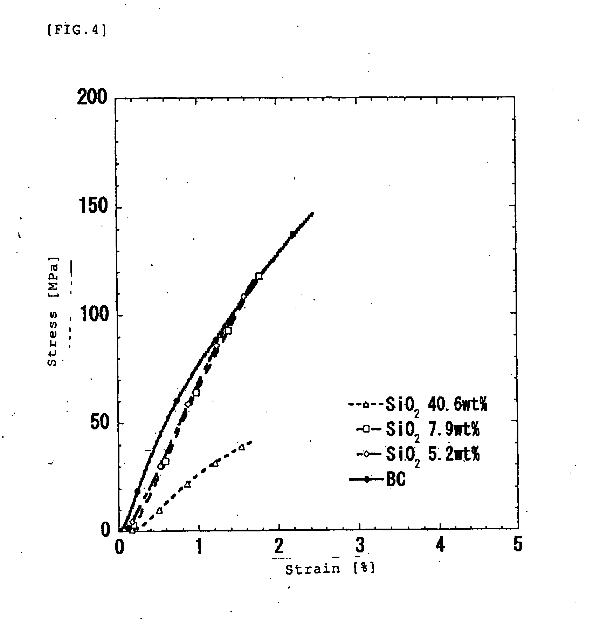

5. Tensile test
   The results are shown in FIG. 4. It is known from the figure that the breaking strength was lowered comparing with bacterial cellulose containing no colloidal silica.

6. DMA test
   The results are shown in FIG. 5. It is known from the figure that the storage modulus was improved comparing with bacterial cellulose containing no colloidal silica.

### (Example 8) Production method of bacterial cellulose in which silas balloon is incorporated

1. Culturing bacterial cellulose producing bacteria in the presence of silas balloon (manufactured by Public Strategy Inc.)
   Culture liquid of 100 ml, mother liquid of 100 ml and silas balloon of 0.1 to 2 g were mixed, developed in a petri dish, and cultured for 25 days.

2. Bleaching of silas balloon bacterial cellulose gel
   After 25 days, the generated gel was sufficiently washed with running water, next immersed in 0.5 wt% aqueous sodium hypochlorite for 12 hours to bleach, then washed sufficiently with running water to yield a silas balloon bacterial cellulose sample.

3 Production of compressed film of silas balloon bacterial cellulose sample
   The silas balloon bacterial cellulose sample was pressed by a heat press at 120°C, at 1 to 2 MPa to yield a film.

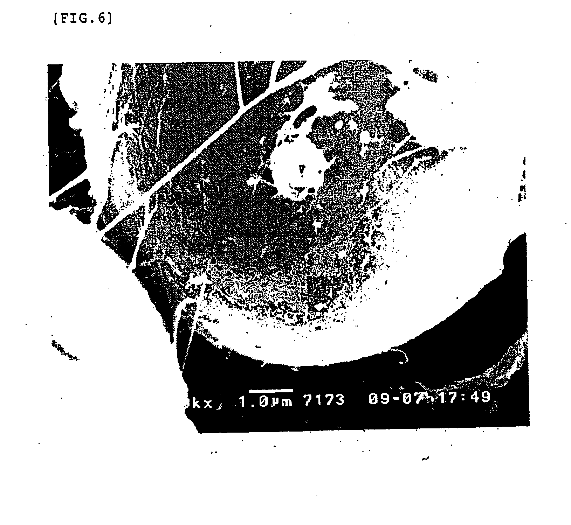

   FIG. 6 is an electron microscope photograph of silas balloon bacterial cellulose, it is known that fibrils of bacterial cellulose are generated on the surface of silas balloon (sphere of about several µm) and the periphery space.

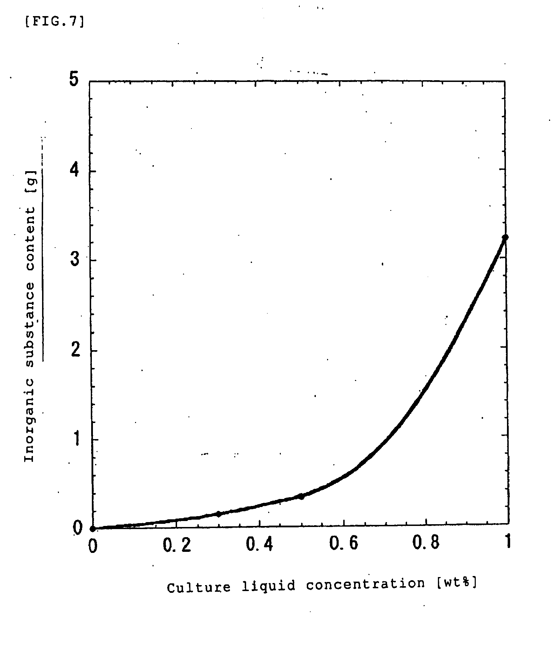

4. Measurement for presence of silas balloon in silas balloon bacterial cellulose sample
   About 100 mg of the dried film of silas balloon bacterial cellulose sample was weighed out, heated at 900°C in an electric furnace for 3 hours, the content of inorganic component was estimated from the weight of ash. The results are shown in FIG. 7. This indicates the presence of silas balloon.

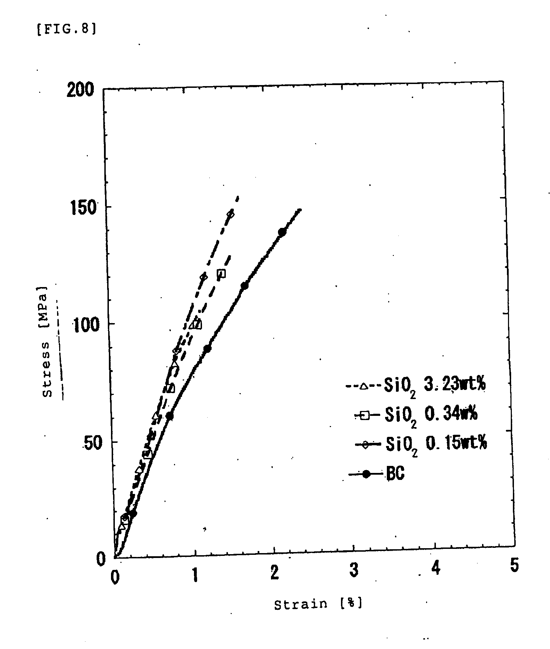

5. Tensile test
   The results are shown in FIG. 8. It is known from the figure that the breaking strength was lowered with an increase in fill of silas balloon comparing with bacterial cellulose containing no silas balloon.

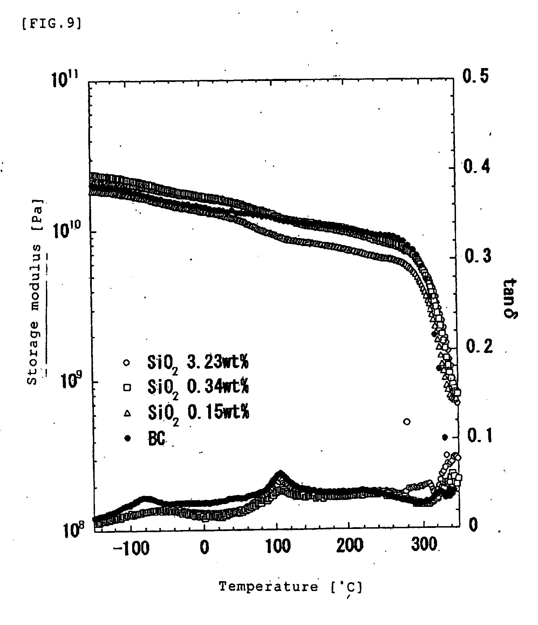

6. DMA test
   The results are shown in FIG. 9. It is known from the figure that the storage modulus was improved below room temperature comparing with bacterial cellulose containing no silica balloon, whereas the storage modulus was lowered above room temperature comparing with bacterial cellulose containing no silica balloon. Also, a tan δ peak became wide with an increase in fill of silas balloon, and shifted to higher temperatures. This is thought that the motion of pyranose ring is restricted by a hydrogen bond between OH of silanol group and OH group on pyranose ring.

### (Example 9) Production method of bacterial cellulose in which carbon nanotube is incorporated

1. Culturing bacterial cellulose producing bacteria in the presence of carbon nanotube (manufactured by Bussan Nanotech Institute Inc.)
   Culture liquid of 100 ml, mother liquid of 100 ml and carbon nanotube of 0.02 to 1 g were mixed, developed in a petri dish, and cultured for 25 days.

2. Bleaching of carbon nanotube bacterial cellulose gel After 25 days, the generated gel was sufficiently washed with running water, next immersed in 0.5 wt% aqueous sodium hypochlorite for 12 hours to bleach, then washed sufficiently with running water to yield a carbon nanotube bacterial cellulose sample.

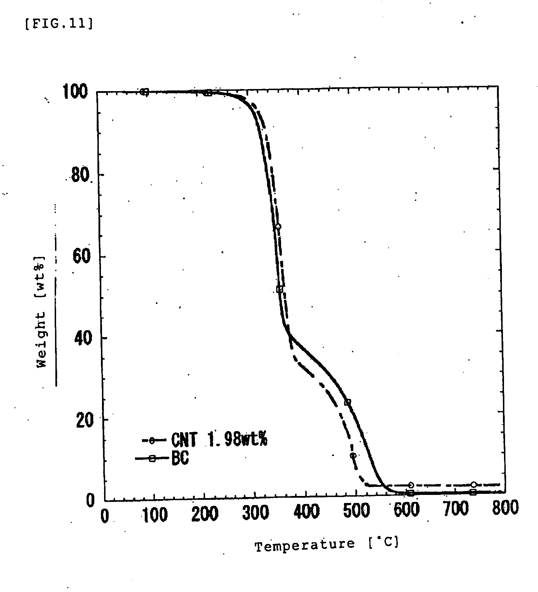

3 Production of compressed film of carbon nanotube bacterial cellulose sample
   The carbon nanotube bacterial cellulose sample was pressed by a heat press at 120°C, at 1 to 2 MPa to yield a film. FIG. 10 is an electron microscope photograph of carbon nanotube bacterial cellulose, it is known that carbon
nanotube (sphere of about several nm) and fibrils of bacterial cellulose are intricately entwined.

4. Measurement for presence of carbon nanotube in carbon nanotube bacterial cellulose sample
   About 100 mg of the dried film of carbon nanotube bacterial cellulose sample was weighed out, heated at 900°C in an electric furnace for 3 hours, the content of inorganic component was estimated from the weight of ash. The results are shown in FIG. 11. This indicates the presence of carbon nanotube.

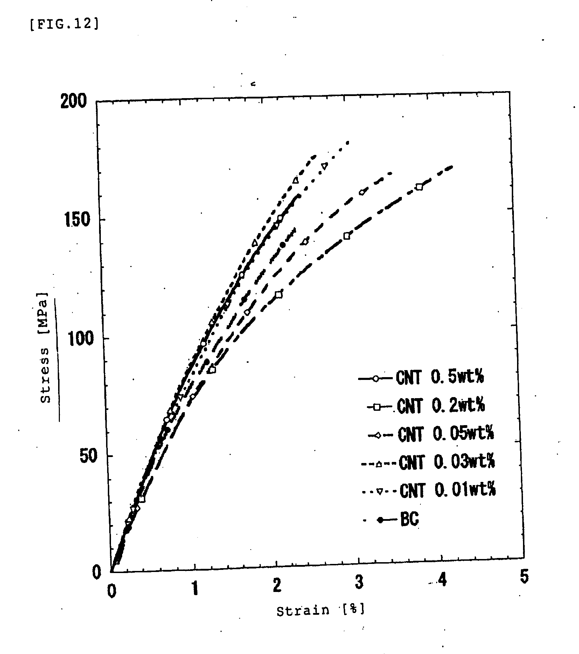

5. Tensile test
   The results are shown in FIG. 12. It is known from the figure that the breaking strength and strain was improved comparing with bacterial cellulose containing no carbon nanotube.

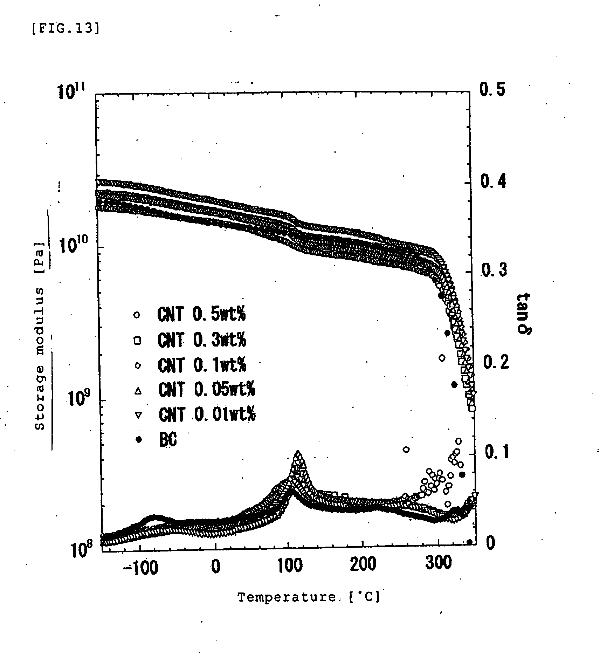

6. DMA test
   The results are shown in FIG. 13. It is known from the figure that both storage modulus and heat stability were improved comparing with bacterial cellulose containing no carbon nanotube.

### (Example 10) Production method of bacterial cellulose in which polyvinyl alcohol is incorporated

1. Culturing bacterial cellulose producing bacteria in the presence of polyvinyl alcohol (manufactured by SCIENTIFIC POLYMER PRODUCTS, INC.)
   Culture liquid of 100 ml, mother liquid of 100 ml and polyvinyl alcohol of 0.1 to 4 g were mixed, developed in a petri dish, and cultured for 25 days.

2. Bleaching of polyvinyl alcohol bacterial cellulose gel After 25 days, the generated gel was sufficiently washed with running water, next immersed in 0.5 wt% aqueous sodium hypochlorite for 12 hours to bleach, then washed sufficiently with running water to yield a polyvinyl alcohol bacterial cellulose sample.

3 Production of compressed film of polyvinyl alcohol bacterial cellulose sample
   The polyvinyl alcohol bacterial cellulose sample was pressed by a heat press at 120°C, at 1 to 2 MPa to yield a film.

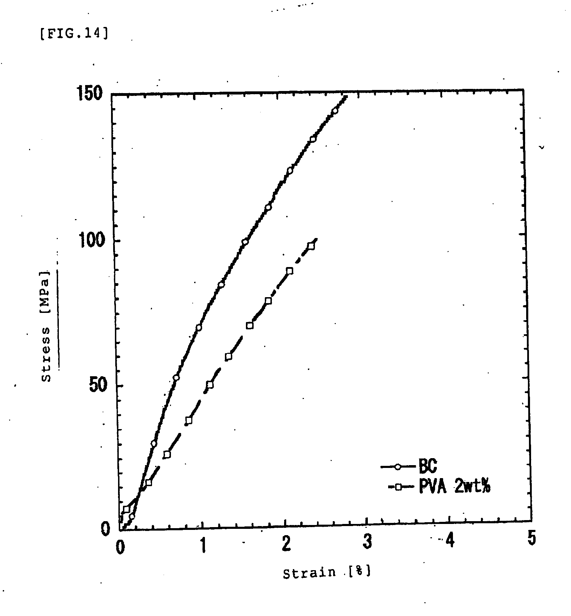

5. Tensile test
   The results are shown in FIG. 14. It is known from the figure that the breaking strength was lowered comparing with bacterial cellulose containing no polyvinyl alcohol.

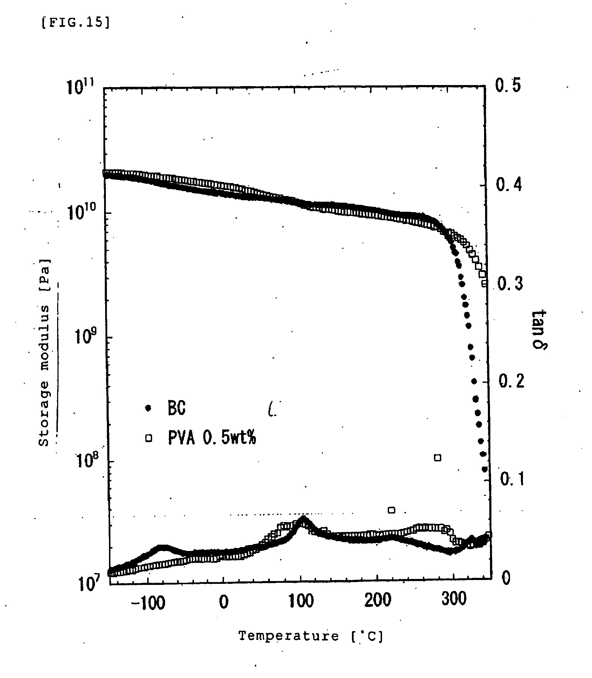

6. DMA test
   The results are shown in FIG. 15. It is known from the figure that the storage modulus was lowered comparing with bacterial cellulose containing no polyvinyl alcohol.

### (Example 11) Production method of bacterial cellulose in which hydroxypropylcellulose is incorporated

1. Culturing bacterial cellulose producing bacteria in the presence of hydroxypropylcellulose (manufactured by Nippon Soda Co. Ltd.)
   Culture liquid of 100 ml, mother liquid of 100 ml and hydroxypropylcellulose of 0.1 to 4 g were mixed, developed in a petri dish, and cultured for 25 days.

2. Bleaching of hydroxypropylcellulose bacterial cellulose gel
   After 25 days, the generated gel was sufficiently washed with running water, next immersed in 0.5 wt% aqueous sodium hypochlorite for 12 hours to bleach, then washed sufficiently with running water to yield a hydroxypropylcellulose bacterial cellulose sample.

3 Production of compressed film of hydroxypropylcellulose bacterial cellulose sample
   The hydroxypropylcellulose bacterial cellulose sample was pressed by a heat press at 120°C, at 1 to 2 MPa to yield a film.

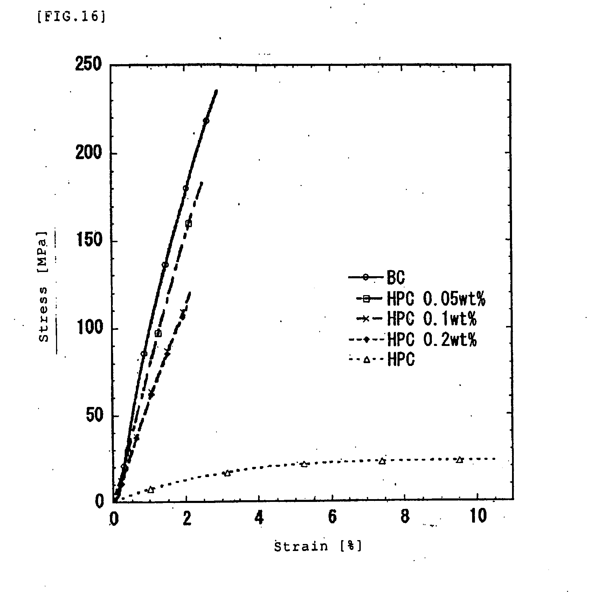

5. Tensile test
   The results are shown in FIG. 16. It is known from the figure that the breaking strength was lowered comparing with bacterial cellulose containing no hydroxypropylcellulose.

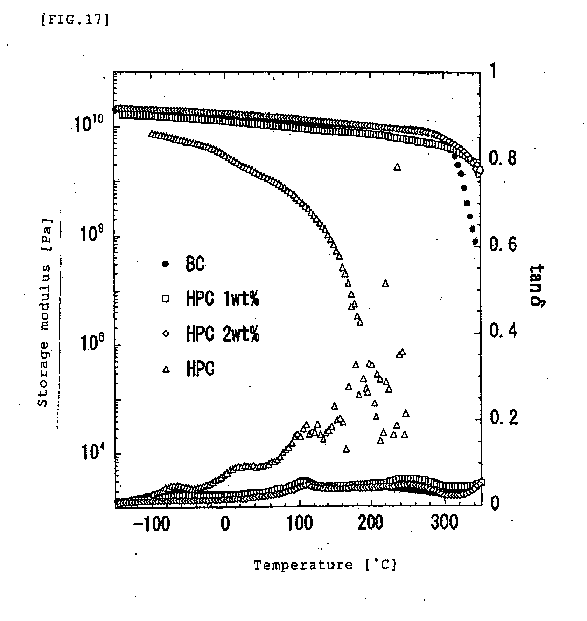

6. DMA test
   The results are shown in FIG. 17. It is known from the figure that the storage modulus was lowered comparing with bacterial cellulose containing no hydroxypropylcellulose.

### (Example 12) Drying of bacterial cellulose gel

About 20 mm× 20 mm× 20 mm (8 g) of bacterial cellulose gel was placed in a stainless steel autoclave of 100 ml in volume. Ethanol was introduced thereto, and held in the condition of about 6.5 MPa and about 243°C. About 3 minutes later, the pressure was returned to ambient pressure, and ethanol was removed. The resultant dried bacterial cellulose gel had almost no change in its shape and weighed 0.5 g. As a result, it is known that the bacterial cellulose gel can be dried almost completely maintaining its shape with a supercritical ethanol.

Also, when the resultant dried bacterial cellulose gel was put into water as it was, left therein, a bacterial cellulose hydrogel was regenerated. Also, the resulting dried bacterial cellulose gel was compressed into a plate, then put in a warm water and left, similarly a bacterial cellulose hydrogel was regenerated. This indicates that even compression of dried bacterial cellulose gel does not cause a structure change because of rearrangement of hydroxyl group.

### (Example 13) Production of bacterial cellulose aerogel

A bacterial cellulose gel was washed, cut to a cubic of 10 mm× 10 mm× 10 mm to yield a sample. The resultant sample was immersed in ethanol for 24 hours, then ethanol was renewed, further immersed therein for 24 hours. This procedure was repeated three times, so that the dispersion medium was changed from water to ethanol. The sample was placed in an autoclave of 50 ml, treated under the supercritical condition of ethanol at a pressure of 6.38 MPa and temperature of 243 to 300°C for 10 minutes. The resultant dried bacterial cellulose gel (bacterial aerogel) had a shape of 10 mm× 10 mm× 10 mm. This result indicates that there is almost no change in shape by drying treatment. Also, the weight was 6 mg. It is known from the result that the bacterial cellulose aerogel obtained has a density of about 6 mg/cm³, and it is a very light material.

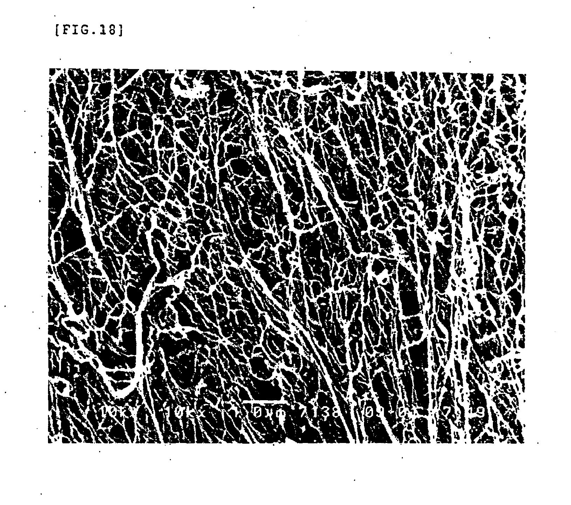

FIG. 18 shows a photograph of scanning electron microscope (SEM) on a cross section of the bacterial cellulose aerogel obtained. It is known that the internal space is filled almost uniformly with a network structure entwined with a lot of fine fibrils. It is also known that the fibril is of nanometer order.

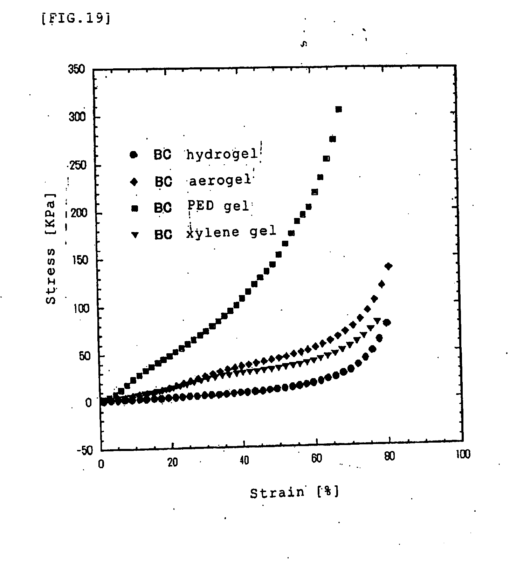

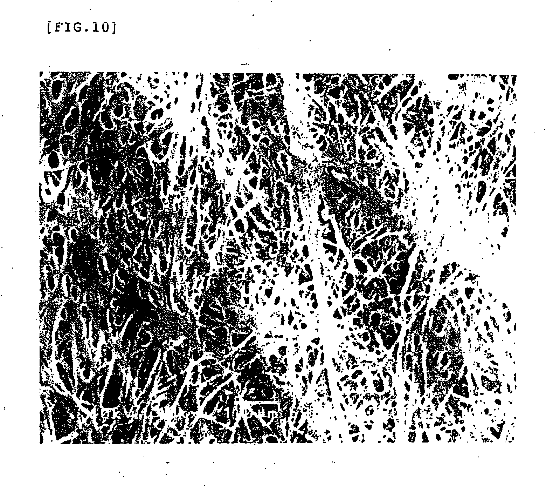

FIG. 19 shows the results of compression strength measured using an almighty tester according to JIS K7208 method. It is known that about twice strength is obtained comparing with hydrogel. This result suggests that hydrogel is plasticized with water.

### (Example 14)

When the aerogel obtained above was contacted with water at room temperature under a vacuum of 11 mmHg, it absorbed water and became a hydrogel. The shape and weight of the resultant hydrogel were 10 mm× 10 mm× 10 mm and 1 g, respectively.

### (Example 15)

When the aerogel obtained above was contacted with the following organic solvents at room temperature under a vacuum of 11 mmHg, it absorbed the solvents and became organogels.
Solvent: xylene, shape of 13.6 mm× 14.0 mm× 12.1 mm, weight of 2.104 g
Solvent: polyethylene oxide, shape of 11.9 mm× 12.12 mm× 7.3 mm, weight of 1.383 g
Also, FIG. 19 shows the results of compression strength measured according to JIS K7208 method. It is known that the strength depends on the kind of solvent. The result is thought to be due to viscosity and polarity of various solvents.

### (Example 16)

By contacting the aerogel obtained above with water in which the following salt was dissolved, or an organic solvent at room temperature under a vacuum of 11 mmHg, a hydrogel in which salt is dispersed, or an organogel was obtained.
Solvent (salt) : polyethylene oxide (LiN(CF₃SO₂)₂), shape of 12.1 mm× 11.6 mm× 11.7 mm, weight of 2.104 g
Also, FIG. 19 shows the result of compression strength measured according to JIS K7208 method. It is known that the strength depends on the kind of solvent. The result is assumed to be due to viscosity of solvent and interaction with cellulose.

### (Example 17) Bacterial cellulose-PEO organogel having Li⁺ conductance

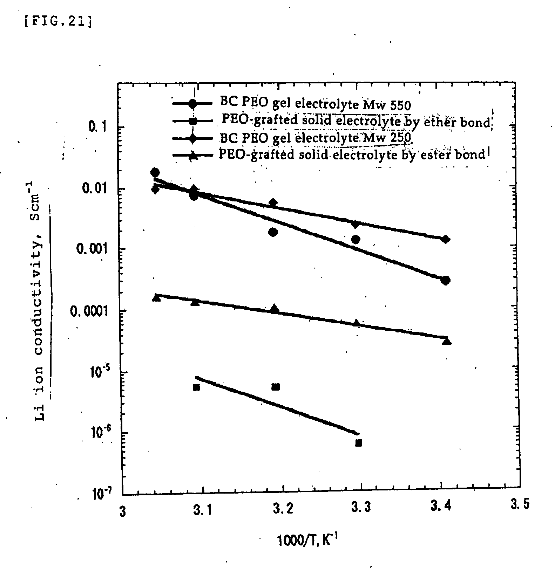

Polyethylene oxide (PEO) with a molecular weight of 250 in which lithium salt was dissolved was added to a bacterial cellulose aerogel under a reduced pressure to give a bacterial cellulose-PEO organogel having Li⁺ conductance. As shown in FIG. 21, the Li⁺ electrolyte showed the almost same Li⁺ conductivity as the lithium salt PEO solution.

Also, FIG. 19 shows the results of compression strength test for bacterial cellulose hydrogel, bacterial cellulose aerogel, and bacterial cellulose-PEO organogel. It is known from the results that the hydrogel is weaker than the aerogel, and the organogel is the strongest.

### (Example 18) Bacterial cellulose PEO ether

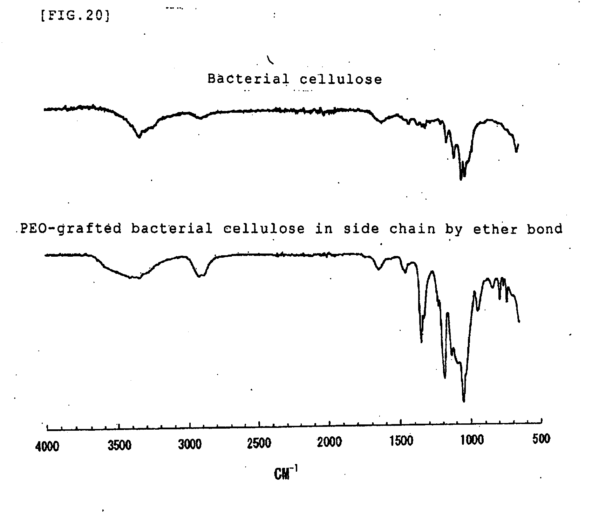

A bacterial cellulose aerogel (10 mm× 10 mm× 10 mm, 6 mg) was reacted with sodium methoxide of 0.027 g in 50 ml of xylene for 1 hour while stirring. Xylene was then removed by a reduced pressure, it was reacted with ethylene oxide of 50 g at 8 MPa, 140°C for 6 hours. The resultant crude reaction product was repeatedly washed with ethanol, water, then acetone to give a bacterial cellulose PEO ether. FIG. 20 shows infrared absorption spectra of the ether having the PEO side chain thus obtained.

### (Example 19) Bacterial cellulose PEO ether Li ion conductive membrane

The bacterial cellulose PEO ether obtained in the above Example was immersed in an ethanol solution of lithium trifluoromethanesulfoneimide (LiTFSI) for 24 hours.
Afterward, it was dried under a reduced pressure at 120°C to give a bacterial cellulose PEO ether Li ion conductive membrane.

FIG. 21 shows the measuring results of lithium ion conductivity for the ion conductive membranes obtained.

### (Example 20) Bacterial cellulose PEO ester

Poly(ethyleneglycol)methyl ether (Mn 350) (PEG-350) of 8.2 g was reacted with Jones reagent (57. 2 g) in 150 ml of acetone while stirring for 48 hours to oxidize the terminal hydroxyl group. The reaction was terminated by adding 20 ml of isopropyl alcohol. Then, the resultant solution was extracted with chloroform, washed with water, and dried to give a terminal carboxylic acid (PEO-350 monocarboxylic acid).

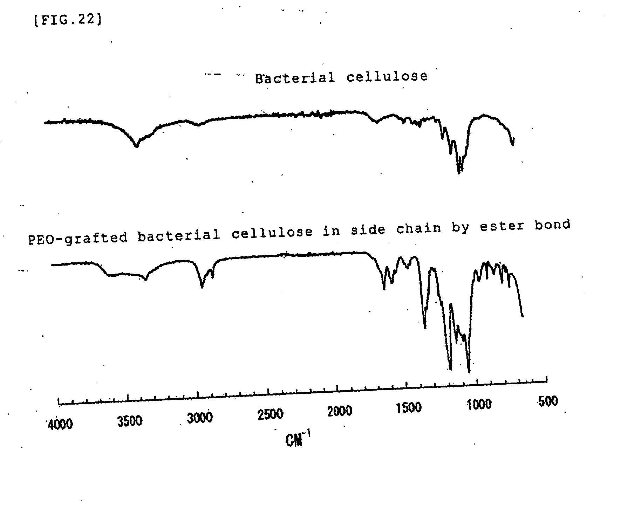

A bacterial cellulose hydrogel (20 mm× 20 mm× 10 mm, 4 g) was held in 200 ml of N,N'-dimethylacetoamide (DMAc) for 24 hours, repeated 5 times to exchange water and DMAc.
Further, dehydrating condensation reaction was conducted at room temperature for 4 days in the presence of the above PEO-350 monocarboxylic acid, 0.2 g of 4-[N,N'-dimethylamino]pyridine (DMAP), and 3.3 g of N,N'-dicyclohexylcarbodiimide (DCC). The resultant crude reaction product was washed with ethanol, water, next acetone to give a bacterial cellulose PEO ester. FIG. 22 shows infrared absorption spectra of the ester having the PEO side chain thus obtained.

### (Example 21) Bacterial cellulose PEO ester Li ion conductive membrane

The bacterial cellulose PEO ester obtained in the above Example was immersed in an ethanol solution of lithium trifluoromethanesulfoneimide (LiTFSI) for 48 hours.
Afterward, it was dried under a reduced pressure at room temperature to give a bacterial cellulose PEO ester Li ion conductive membrane.

FIG. 21 shows the measuring results of lithium ion conductivity for the ion conductive membranes obtained.

### (Example 22) Organic-Inorganic composite aerogel having an IPN structure

To a bacterial cellulose hydrogel (10 mm× 10 mm× 10 mm, 1 g), 17.3 g of tetraethoxysilane in 500 ml of water was added to conduct in situ polymerization. The resultant gel was subjected to ethanol supercritical drying. From the result of scanning electron microscope observation, it is known that the organic-inorganic composite aerogel has an IPN structure.

### (Example 23) Bacterial cellulose aerogel dehydrate

A bacterial cellulose aerogel (30 mm× 20 mm× 15 mm, 52 mg) was placed in a round bottom flask, being reduced pressure by a vacuum pump, heat-dehydration was conducted at 350°C under 0.1 mmHg for 4 hours to give a black sponge-like dehydrate of bacterial cellulose aerogel of 1.7 mg.

### (Example 24) Production of cathode and lithium battery

In a mortar were placed 4 g of LiMn₂O₄ powder, 0.75 g of graphite and 0.5 g of 5 wt% polyvinylidene fluoride/N-methylpyrrolidone solution, and kneaded. The kneaded product was spread on a teflon sheet and dried in a dryer at 100°C for 1 hour. It was covered with a stainless mesh, pressed at room temperature under 3 t/cm² for pressure adhesion to give a cathode. BC gel electrolyte was sandwiched with this cathode and a lithium foil to give a battery. Voltage of 6 V was applied to the battery, being charged for 30 minutes. The voltage of this battery was 3.4 V.

### Industrial Applicability

The material of the present invention is a lithium ion conductive material using a novel material, can easily construct a lithium ion battery. Accordingly, it can be widely used in various technical fields utilizing the lithium ion conductive material and lithium ion battery, for example, home appliances, electronic devices, automobiles, buildings, optical apparatuses, aerospace-related apparatuses and other markets.

The bacterial cellulose composite material of the present invention has a structure that an inorganic material and/or an organic material are incorporated in bacterial cellulose. Therefore, such material exhibits excellent moldability, mechanical and electrical characteristics, and biodegradability. The effects resulted from such novel material are unpredictable from conventionally known materials and extremely excellent properties, which can solve many of unsolved problems that have been strongly asked to solve by conventional composite materials. In various technical fields, for example, drugs and medicines, medical products, medical device, home appliances, electronic devices, automobiles, buildings, optical apparatuses, aerospace-related apparatuses and other markets, the material of the present invention having novel properties meets very large demands, so that the industrial applicability is extremely high.

The bacterial cellulose aerogel of the present invention has excellent filter performance, absorption ratio, absorption velocity and liquid permeation, also excellent storage stability and strength of gel after absorption of water. The material also has absorption capability of organic solvents. Thus the material is useful for sanitary materials such as sanitary napkin, paper diaper, sheet for adult, tampon and sanitary cotton. Also, the above-mentioned material is used for a long time without deterioration of its gel structure, further is quite flexible, so that it can be used for materials for gardening, soil and building such as water retention agent and water-shutting agent. Furthermore, the above high water absorption polymer is expected to have applications for cosmetics emphasizing shape, elasticity, water absorption and air permeation.

## Claims

1. A lithium ion conductive material wherein water in a bacterial cellulose hydrogel is replaced by a nonaqueous solvent containing a lithium compound.

2. The lithium ion conductive material of claim 1, wherein the nonaqueous solvent is selected from the group consisting of polyethylene glycol dimethyl ether, polyethylene glycol diethyl ether, polyethylene glycol dimethacrylate, polyethylene glycol diacrylate, polypropylene glycol dimethacrylate, and polypropylene glycol diacrylate.

3. The lithium ion conductive material of claim 2, wherein the nonaqueous solvent is polyethylene glycol dimethyl ether.

4. The lithium ion conductive material of any one of claims 1 to 3, wherein the lithium compound is selected from the group consisting of lithium perchlorate (LiClO₄), lithium borate tetrafluoride (LiBF₄), lithium phosphate hexafluoride (LiPF₆), lithium methanesulfonate trifluoride (LiCF₃SO₃), and lithium bistrifluoromethanesulfonylimide (LiN(CF₃SO₂)₂).

5. The lithium ion conductive material of claim 4, wherein the lithium compound is lithium trifluoromethanesulfoneimide.

6. A production method of a lithium ion conductive material, comprising the steps of:
immersing a bacterial cellulose hydrogel in a nonaqueous solvent containing a lithium compound; being allowed to stand for a certain time under a reduced pressure and
heating; subsequently raising temperature and further being allowed to stand for a certain time under a reduced pressure to exchange dispersion media.

7. The method of claim 6, wherein the nonaqueous solvent is selected from the group consisting of polyethylene glycol dimethyl ether, polyethylene glycol diethyl ether, polyethylene glycol dimethacrylate, polyethylene glycol diacrylate, polypropylene glycol dimethacrylate, and polypropylene glycol diacrylate.

8. The method of claim 7, wherein the nonaqueous solvent is polyethylene glycol dimethyl ether.

9. The method of any one of claims 6 to 8, wherein the lithium compound is selected from the group consisting of lithium perchlorate (LiClO₄), lithium borate tetrafluoride (LiBF₄), lithium phosphate hexafluoride (LiPF₆), lithium methanesulfonate trifluoride (LiCF₃SO₃), and lithium bistrifluoromethanesulfonylimide (LiN(CF₃SO₂)₂).

10. The method of any one of claims 6 to 9, wherein the heating temperature and standing time at the first step are 30 to 90°C and 12 to 36 hours, respectively; the heating temperature and standing time at the second step are 100 to 160°C and 12 to 36 hours, respectively.

11. The method of claim 10, wherein the heating temperature and standing time at the first step are 60°C and 24 hours, respectively; the heating temperature and standing time at the second step are 130°C and 24 hours, respectively.

12. A lithium ion battery comprising a cathode, an anode and the lithium ion conductive material of any one of claims 1 to 5 being disposed between the cathode and the anode.

13. A bacterial cellulose composite material wherein an inorganic material and/or an organic material are incorporated.

14. The composite material of claim 13, wherein the inorganic material and/or the organic material are silica gel, silas balloon, carbon nanotube and/or polyvinyl alcohol, hydroxypropylcellulose.

15. A production method of a bacterial cellulose composite material wherein an inorganic material and/or an organic material are incorporated, wherein a bacterial cellulose producing bacterium is cultured in a culture medium added with an inorganic material and/or an organic material.

16. The production method of claim 15, wherein in the culture medium, as a carbon source, glucose, mannitol, sucrose, maltose, hydrolyzed starch, molasses, ethanol, acetic acid, or citric acid is used; as a nitrogen source, ammonium salt such as ammonium sulfate, ammonium chloride, and ammonium phosphate, nitrate, urea, or polypeptone is used; as inorganic salts, phosphate, calcium salt, iron salt or manganese salt is used; and as an organic trace nutrient, amino acid, vitamin, fatty acid, nucleic acid, casamino acid, yeast extract, or hydrolyzed soy protein is used.

17. The method of claim 15 or claim 16, wherein the culture medium includes glucose, polypeptone, yeast extract, and mannitol.

18. The method of any one of claims 15 to 17, wherein the bacterial cellulose producing bacterium is a microbe belonging to Acetobacter, Gluconobacter, Agrobacterium or Pseudomonas.

19. The method of any one of claims 15 to 18, wherein the bacterial cellulose producing bacterium is Acetobacter xylinum.

20. The method of any one of claims 15 to 19, wherein the inorganic material and/or the organic material are silica gel, silas balloon, carbon nanotube and/or polyvinyl alcohol, hydroxypropylcellulose.

21. A bacterial cellulose aerogel.

22. A production method of bacterial cellulose aerogel, wherein a bacterial cellulose hydrogel is dehydrated and dried with a supercritical ethanol.

23. A production method of bacterial cellulose hydrogel, wherein water or water containing a salt is absorbed in a bacterial cellulose aerogel.

24. A production method of bacterial cellulose organogel wherein an organic solvent or a solvent containing a salt is absorbed in a bacterial cellulose aerogel.
